# EUROPEAN PATENT SPECIFICATION

(11) **EP 3 351 237 B1**
(45) Date of publication and mention of the grant of the patent: **18.12.2024**
(21) Application number: 18159139.7
(22) Date of filing: 02.11.2012
(51) Int. Cl.: A61K 8/65, A61K 8/73, A61Q 19/00, A61Q 19/08, A61L 27/52

(54) **HYALURONIC ACID-COLLAGEN MATRICES FOR DERMAL FILLING AND VOLUMIZING APPLICATIONS**
HYALURONSÄURE-KOLLAGEN-MATRIZEN FÜR DERMALE FÜLLUNG UND VOLUMENGEBENDE ANWENDUNGEN
MATRICES D'ACIDE HYALURONIQUE-COLLAGÈNE POUR DES APPLICATIONS DE VOLUMISATION ET DE REMPLISSAGE DERMIQUE

(30) Priority: 04.11.2011 US 201161555970 P; 04.09.2012 US 201213603213; 06.09.2012 US 201213605565
(43) Date of publication of application: 25.07.2018
(62) Divisional of application: 12783840.7
(73) Proprietor: Allergan, Inc., Irvine, CA 92612 (US)
(72) Inventor: YU, Xiaojie, Irvine, CA California 92602 (US); Manesis, Nicholas J., San Diego CA California 92198 (US); Pollock, Jacob F., Charleston, WV West Virginia 25314 (US)
(74) Representative: Hoffmann Eitle

(56) References cited:
- EP-A1- 1 419 792
- WO-A1-2010/053918
- WO-A1-2011/119468
- WO-A2-2010/003104
- PARK S-N ET AL: "Characterization of porous collagen/hyaluronic acid scaffold modified by 1-ethyl-3-(3-dimethylaminopropyl)carbodiimide cross-linking", BIOMATERIALS, ELSEVIER SCIENCE PUBLISHERS BV., BARKING, GB, vol. 23, no. 4, 15 February 2002 (2002-02-15), pages 1205 - 1212, XP004348139, ISSN: 0142-9612, DOI: 10.1016/S0142-9612(01)00235-6
- PARK S ET AL: "Biological characterization of EDC-crosslinked collagen-hyaluronic acid matrix in dermal tissue restoration", BIOMATERIALS, ELSEVIER SCIENCE PUBLISHERS BV., BARKING, GB, vol. 24, no. 9, 1 April 2003 (2003-04-01), pages 1631 - 1641, XP004404219, ISSN: 0142-9612, DOI: 10.1016/S0142-9612(02)00550-1

## Description

This application claims priority to U.S. Provisional Patent Application No. 61/555,970, filed November 4, 2011, and also is a continuation-in-part of U.S. Patent Application Serial No. 13/605,565, filed on September 6, 2012, which claims priority to U.S. Provisional Patent Application No. 61/531,533, filed on September 6, 2011, and which is a continuation-in-part of U.S. Patent Application No. 13/603,213, filed September 4, 2012, which claims priority to U.S. Provisional Patent Application No. 61/531,533, filed September 6, 2011.

This application generally relates to biocompatible, implantable compositions and more specifically relates to hyaluronic acid-collagen based compositions useful as dermal fillers as defined in the claims. WO 2011/119468 A1 discloses polysaccharide-protein hydrogels for soft tissue augmentation. WO 2010/003104 A2 discloses kits for use in filling and regenerating tissue comprising instructions for use.

### SUMMARY

The present invention generally relates to a soft tissue aesthetic product as defined in the claims. In one aspect, the product comprises a filler comprising a homogeneous hydrogel having a form suitable for injecting into human tissue; and a label comprising instructions to inject the filler into the human tissue; wherein the homogeneous hydrogel comprises water, and a crosslinked macromolecular matrix as defined in claim 1.

Some embodiments include a method of improving an aesthetic quality of soft tissue of a human being comprising: injecting a homogeneous hydrogel composition into a soft tissue of the human being to thereby improve the aesthetic quality of the soft tissue; wherein the homogeneous hydrogel composition comprises water, and a crosslinked macromolecular matrix as defined in claim 6.

The crosslinked molecular matrices according to the invention comprise a hyaluronic acid component; and a collagen component; wherein the hyaluronic acid component is crosslinked to the collagen component by a crosslinking component; and wherein the crosslinking component comprises a plurality of crosslink units, wherein at least a portion of the crosslink units comprise an ester bond or an amide bond as defined in the claims.

### BRIEF DESCRIPTION OF THE DRAWINGS

Some aspects of the present disclosure may be more clearly understood with reference to the appended drawings of which:
FIG.1 is a plot of frequency sweep and strain sweep for a hydrogel in accordance with this invention.
FIG. 2 is an extrusion profile through a 30G needle for a hydrogel from Example 4.

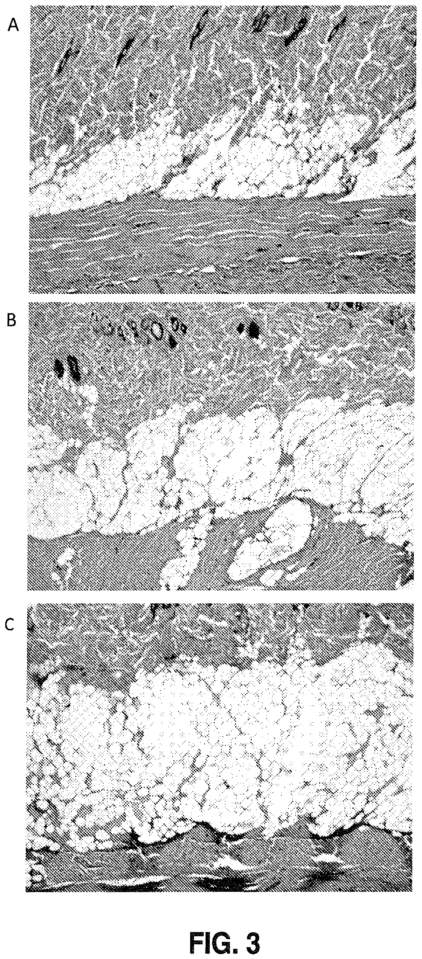

FIG. 3 shows micrographs (at 5X magnification) of (A) tissue adjacent to an implanted control composition of commercial crosslinked hyaluronic acid gel, (B) tissue adjacent to an implanted composition of Example 3, and (C) tissue adjacent to an implanted composition from Example 4.

### DETAILED DESCRIPTION

Embodiments according to the invention include a method of improving an aesthetic quality of soft tissue of a human as defined in the claims. Such a method comprises injecting a homogeneous hydrogel composition, or a composition comprising a homogeneous hydrogel, into a soft tissue of the human being to thereby improve the aesthetic quality of the soft tissue.

Embodiments according to the invention include a soft tissue aesthetic product comprising: a filler comprising a homogeneous hydrogel having a form suitable for injecting into human tissue and a label comprising instructions to inject the filler into the human tissue as defined in the claims.

A filler comprising a homogeneous hydrogel or a hydrogel composition may be any kind of filler as defined in the claims that is suitable for injecting into human tissue to improve an aesthetic quality of a soft tissue, such as a dermal filler, a breast augmentation or reconstruction filler, a lip filler, hand rejuvenation, or the like. When injected, a hydrogel may stimulate tissue in-growth and generation after being injected into the soft tissue. In some embodiments, a hydrogel may stimulate collagenesis after being injected into the soft tissue.

Injecting a homogenous hydrogel comprising a crosslinked macromolecular matrix comprising a hyaluronic acid component that is crosslinked to a collagen component as defined in the claims may provide improved aesthetic quality for an extended duration, as compared to injecting an identical hydrogel except that hyaluronic acid component and the collagen component are not crosslinked.

A packaged product is described which comprises a syringe loaded with a hydrogel and a needle. A syringe may be fitted with a needle of any size that is appropriate for injecting the hydrogel into the soft tissue of interest, such as a needle with about a #25, about a #30, or a larger gauge.

A filler comprising a hydrogel may be suitable for injection if it can be injected into the soft tissue of interest without unreasonable difficulty, and includes fillers that can be dispensed from cannulas having gauge as low as about #30 or about #25 under normal manual pressure with a smooth extrusion plateau.

Injection of a homogenous hydrogel in accordance with the present invention may provide a soft tissue augmentation that mimics the natural components of the skin. A homogenous hydrogel may be injected intradermally or subcutaneously to augment soft tissue and to repair or correct congenital anomalies, acquired defects, or cosmetic defects. Examples of such conditions include congenital anomalies such as hemifacial microsomia, malar and zygomatic hypoplasia, unilateral mammary hypoplasia, pectus excavatum, pectoralis agenesis (Poland's anomaly), and velopharyngeal incompetence secondary to cleft palate repair or submucous cleft palate (as a retropharyngeal implant); acquired defects (post traumatic, post surgical, or post infectious) such as depressed scars, subcutaneous atrophy (e.g., secondary to discoid lupis erythematosis), keratotic lesions, enopthalmos in the unucleated eye (also superior sulcus syndrome), acne pitting of the face, linear scleroderma with subcutaneous atrophy, saddle-nose deformity, Romberg's disease, and unilateral vocal cord paralysis; and cosmetic defects such as glabellar frown lines, deep nasolabial creases, circum-oral geographical wrinkles, sunken cheeks, and mammary hypoplasia.

Crosslinked hyaluronic acid, collagen, and crosslinked collagen, such as those used in dermal fillers, do not promote cellular infiltration and tissue in-growth. Similarly, collagen simply blended into hyaluronic acid hydrogels does not promote tissue integration or *de novo* tissue generation. However, the hydrogels as defined in the claims do promote cellular migration into the hydrogels and tissue formation within the gels when implanted *in vivo.*

A hydrogel may comprise water and a crosslinked macromolecular matrix as defined in the claims. Typically, a crosslinked molecular matrix may comprise a hyaluronic acid component and a collagen component, wherein the hyaluronic acid component is crosslinked to the collagen component by a crosslinking component. A crosslinking component may comprise a plurality of crosslink units, wherein at least a portion of the crosslink units comprise an ester bond or an amide bond as set out in the claims.

A crosslinked macromolecular matrix for a hydrogel may be synthesized by coupling a hyaluronic acid with a collagen using a coupling agent, such as a carbodiimide. In these hydrogels, hyaluronic acid may serve as a biocompatible water-binding component, providing bulk and isovolumetric degradation. Additionally, collagen may impart cell adhesion and signaling domains to promote cell attachment, migration, and other cell functions such as extra-cellular matrix deposition. The biopolymers form homogeneous hydrogels with tunable composition, swelling, and mechanical properties. Compositions can be made to be injectable for minimally invasive implantation through syringe and needle.

Hyaluronic acid is a non-sulfated glycosaminoglycan that enhances water retention and resists hydrostatic stresses. It is non-immunogenic and can be chemically modified in numerous fashions. Hyaluronic acid may be anionic at pH ranges around or above the pKa of its carboxylic acid groups.

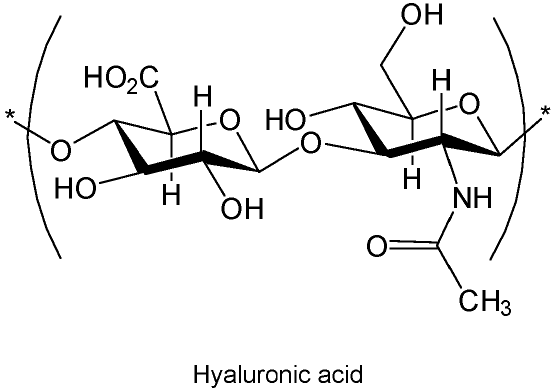

Collagen is a protein that forms fibrils and sheets that bear tensile loads. Collagen also has specific integrin-binding sites for cell adhesion and is known to promote cell attachment, migration, and proliferation. Collagen may be positively charged because of its high content of basic amino acid residues such as arginine, lysine, and hydroxylysine.

Because hyaluronic acid may be anionic and collagen may be cationic, the two macromolecules may form polyionic complexes in aqueous solution. A polyionic complex may be significantly less soluble in water than either hyaluronic acid or collagen, and thus may precipitate out of aqueous solution when the two macromolecules are together in a mixture.

Under certain conditions, a hyaluronic acid and a collagen may be combined in an aqueous liquid in which both components are soluble. A hyaluronic acid and a collagen may then be crosslinked while both are dissolved in an aqueous solution to form a hydrogel. Reaction conditions such as the concentration of hyaluronic acid, the concentration of collagen, the pH of the solution, and salt concentration may be adjusted to help to prevent polyionic complex formation between anionic hyaluronic acid and cationic collagen. They may also help to prevent collagen microfibril formation.

Some embodiments include a method of crosslinking hyaluronic acid and collagen. This method generally comprises a dissolution step which results in an aqueous pre-reaction solution. In a dissolution step, hyaluronic acid and collagen are dissolved in an aqueous solution that has a low pH and/or a salt to form an aqueous pre-reaction solution.

A hyaluronic acid-collagen crosslinking method further comprises an activation step. In an activation step, an aqueous pre-reaction solution is modified at least by adding a water soluble coupling agent and/or by increasing the pH of the solution. If needed, a salt may also be added to keep the hyaluronic acid and collagen in solution at the higher pH. Thus, a crosslinking reaction mixture comprises hyaluronic acid and collagen dissolved or dispersed in an aqueous medium, a water soluble coupling agent, and a salt, and has a higher pH than the aqueous pre-reaction solution from which it was derived. The crosslinking reaction mixture is allowed to react to thereby crosslink the hyaluronic acid and the collagen.

In some embodiments, the pH of the aqueous pre-reaction solution may be increased and a substantial amount of fiber formation may be allowed to occur in the solution before adding the water soluble coupling agent. In some embodiments, the water soluble coupling agent may be added to the aqueous pre-reaction solution before substantially any fiber formation occurs.

A crosslinking reaction mixture can react to form a crosslinked macromolecular matrix. Since reaction occurs in an aqueous solution, a crosslinked macromolecular matrix may be dispersed in an aqueous liquid in hydrogel form as it is formed by a crosslinking reaction. A crosslinked macromolecular matrix may be kept in hydrogel form because, in many instances, a crosslinked macromolecular matrix may be used in hydrogel form.

In some embodiments, an aqueous pre-reaction solution or a crosslinking reaction mixture may further comprise about 10% to about 90% of an organic solvent in which hyaluronic acid has poor solubility, such as ethanol, methanol, isopropanol, or the like.

After a crosslinking reaction has occurred, the crosslinked macromolecular matrix may be particulated or homogenized through a mesh. This may help to form an injectable slurry or hydrogel. A mesh used for particulating a crosslinked macromolecular matrix may have any suitable pore size depending upon the size of particles desired. In some embodiments, the mesh may have a pore size of about 10 microns to about 100 microns, about 50 microns to about 70 microns, or about 60 microns.

A hydrogel comprising a crosslinked molecular matrix may be treated by dialysis for sterilization or other purposes. Dialysis may be carried out by placing a semipermeable membrane between the hydrogel and another liquid so as to allow the hydrogel and the liquid to exchange molecules or salts that can pass between the membrane.

A dialysis membrane may have a molecular weight cutoff that may vary. For example, the cutoff may be about 5,000 daltons to about 100,0000 daltons, about 10,000 daltons to about 30,000 daltons, or about 20,000 daltons.

The dialysis may be carried out against a buffer solution, or the liquid on the other side of the membrane from the hydrogel may be a buffer solution. In some embodiments, the buffer solution may be a sterile phosphate buffer solution that may comprise phosphate buffer, potassium chloride, and/or sodium chloride. A sterile phosphate buffer solution may be substantially isosmotic with respect to human physiological fluid. Thus, when dialysis is complete, the liquid component of a hydrogel may be substantially isosmotic with respect to human physiological fluid.

In some embodiments, a crosslinked macromolecular complex may further comprise an aqueous liquid. For example, the crosslinked macromolecular complex may absorb the aqueous liquid so that a hydrogel is formed. An aqueous liquid may comprise water with a salt dissolved in it, such as a phosphate buffer, sodium chloride, potassium chloride, etc. In some embodiments, an aqueous liquid may comprise water, sodium chloride at a concentration of about 100 mM to about 200 mM, potassium chloride at a concentration of about 2 mM to about 3 mM, and phosphate buffer at a concentration of about 5 mM to about 15 mM, wherein the pH of the liquid is about 7 to about 8.

A hydrogel may be used in a soft tissue aesthetic product as defined in the claims. A soft tissue aesthetic product may comprise: an aesthetic device having a form suitable for injecting or implanting into human tissue; and a label comprising instructions to inject or implant the aesthetic component into human tissue; wherein the aesthetic device comprises a crosslinked macromolecular matrix as defined herein. Some products may comprise the crosslinked macromolecular matrix in hydrogel form.

The hydrogel of a crosslinked macromolecular complex has storage modulus of about 50 Pa to 10,000 Pa such as about 500 Pa to about 1000 Pa, about 556 Pa, about 560 Pa, about 850 Pa, about 852 Pa, or any value in a range bounded by, or between, any of these values.

In some embodiments, a hydrogel of a crosslinked macromolecular complex may have a loss modulus of about 1 Pa to about 500 Pa, about 10 Pa to 200 Pa, about 100 Pa to about 200 Pa, about 20 Pa, about 131 Pa, about 152 Pa, or any value in a range bounded by, or between, any of these values.

In some embodiments, a hydrogel of a crosslinked macromolecular complex may have an average extrusion force of about 20 N to 30 N, or about 25 N, when the hydrogel is forced through a 30G needle syringe by moving the plunger of a 1 mL syringe containing the hydrogel at a rate of 100 mm/min for about 11 mm, and measuring the average force from about 4 mm to about 10 mm.

A crosslinked macromolecular complex may have tunable swelling properties based on reaction conditions and hydrogel dilution. In some embodiments, a crosslinked macromolecular complex may have a swelling ratio of about 1 to about 7. A swelling ratio is the ratio of the weight of the crosslinked macromolecular complex when saturated with water to the weight of the crosslinked macromolecular complex without any water. More specifically, the swelling ratio is the ratio of the mass of the gel which has been allowed to fully swell to the mass of the gel at its initial concentration.

In a crosslinking reaction, the molecular weight of a hyaluronic acid may vary. In some embodiments, a hyaluronic acid may have a molecular weight of about 500,000 daltons to about 10,000,000 daltons, about 1,000,000 daltons to about 5,000,000 daltons, or about 1,000,000 daltons to about 3,000,000 daltons. When the crosslinking reaction occurs, the resulting crosslinked macromolecular product may have a hyaluronic acid component derived from the hyaluronic acid in the crosslinking reaction. Thus, the ranges recited above may also apply to the molecular weight of a hyaluronic acid component, e.g. about 500,000 daltons to about 10,000,000 daltons, about 1,000,000 daltons to about 5,000,000 daltons, or about 1,000,000 daltons to about 3,000,000 daltons. The term "molecular weight" is applied in this situation to a portion of the matrix even though the hyaluronic acid component may not actually be a separate molecule due to the crosslinking.

The concentration of hyaluronic acid in an aqueous pre-reaction solution or a crosslinking reaction mixture may vary. Hyaluronic acid is present at about 6 mg/mL to about 24 mg/mL, such as about 6 mg/mL, about 12 mg/mL, about 16 mg/mL, or about 24 mg/mL

Any type of collagen may be used in the methods and compositions described herein. In some embodiments, collagen type I, collagen type III, collagen type IV, collagen type VI, or a combination thereof, may be used. In some embodiments, a collagen or a collagen component comprises collagen type I or collagen type III. In some embodiments, the collagen component comprises collagen type V.

A collagen may be derived from cell culture, animal tissue, or recombinant means, and may be derived from human, porcine, or bovine sources. Some embodiments comprise collagen derived from human fibroblast culture. Some embodiments comprise collagen that has been denatured to gelatin. The source and/or collagen extraction/processing conditions can alter the way in which collagen macromolecules bundle together to form supramolecular structures. These higher order structures can have effects on the gel physical properties (stiffness, viscosity) and may also have an effect on the reactivity of the collagen to crosslinking reagents.

Collagen concentration in an aqueous pre-reaction solution or a crosslinking reaction mixture may vary. Collagen is present at a concentration of about 1 mg/mL to about 15 mg/mL, such as about 3 mg/mL to about 12 mg/mL, about 1.7 mg/mL, about 3 mg/mL, about 6 mg/mL, about 8 mg/mL, or about 12 mg/mL. The collagen concentration has an effect on the physical properties of the gel (stiffness, viscosity). In general, higher collagen concentrations lead to a higher elastic modulus.

In some embodiments, the weight ratio of hyaluronic acid to collagen in a aqueous pre-reaction solution or a aqueous pre-reaction solution or a crosslinking reaction mixture (e.g. [wt hyaluronic acid]/[wt collagen]) may be about 0.5 to about 3, about 1 to about 3, about 1 to about 2, about 1, or about 2. When the crosslinking reaction occurs, the resulting crosslinked macromolecular product may have a collagen component derived from the collagen in the crosslinking reaction. Thus, the resulting crosslinked macromolecular matrix may have a weight ratio of hyaluronic acid component to collagen component that corresponds to the weight ratio in the crosslinking reaction, e.g. about 0.5 to about 3, about 1 to about 3, about 1 to about 2, about 1, or about 2.

In other embodiments of the invention, the compositions have an HA to collagen ratio of between about 0.5 to 1 and about 7 to 1.

A salt may help to screen the negative charges of hyaluronic acid from positive charges of collagen, and may thus prevent precipitation of a polyionic ion complex from solution. However, high concentrations of salt may reduce the solubility of some components in solution. Thus, in some embodiments, the salt concentration of an aqueous pre-reaction solution or a crosslinking reaction mixture may be high enough to screen the charges so that the polyionic ion complex is not formed, but also low enough so that the components of the mixture remain in solution. For example, the total salt concentration of some aqueous pre-reaction solutions or crosslinking reaction mixtures may be about 10 mM to about 1 M, for example, between about 5 mM to about 0.5 M, for example, between about 2 mM to about 0.2 M.

Some salts in an aqueous pre-reaction solution or a crosslinking reaction mixture may be non-coordinating buffers. Any non-coordinating buffer may be used that is capable of buffering the mixture and does not coordinate with metal atoms or ions in the collagen. In some embodiments, the buffer does not react with the crosslinking reagents (carbodiimide and additive). For example, acetate or phosphate buffers may not be used in these embodiments. Examples of suitable non-coordinating buffers may include, but are not limited to, 2-(N-morpholino)ethanesulfonic acid (MES), 3-(N-morpholino)propanesulfonic acid (MOPS), 4-(2-hydroxyethyl)-1-piperazinyl)ethanesulfonic acid (HEPES), 3-[4-(2-hydroxyethyl)-1-piperazinyl]propanesulfonic acid (HEPPS), N-cyclohexyl-2-aminoethanesulfonic acid (CHES), N-cyclohexyl-3-aminopropanesulfonic acid (CAPS), etc.

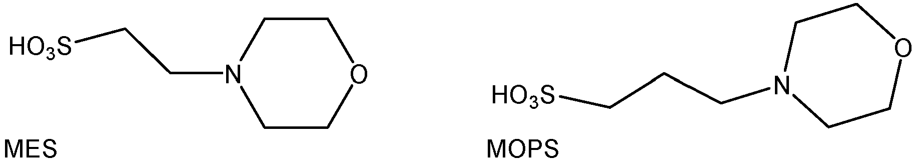

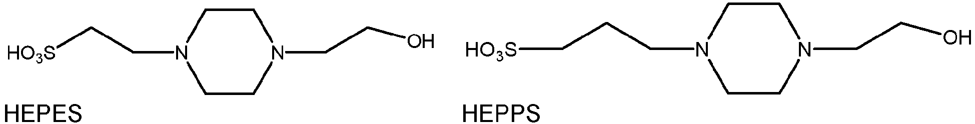

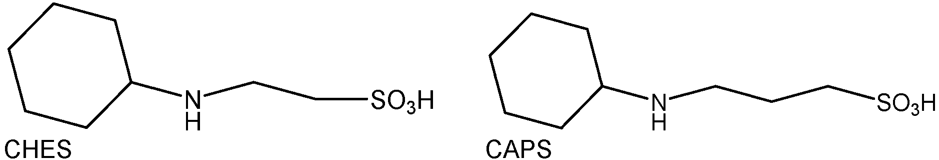

The concentration of a non-coordinating buffer may vary. For example, some aqueous pre-reaction solutions or crosslinking reaction mixtures may have a buffer concentration in a range of about 10 mM to about 1 M, about 10 mM to about 500 mM, about 20 mM to about 100 mM, or about 25 mM to about 250 mM. Some aqueous pre-reaction solutions or crosslinking reaction mixtures comprise MES at a concentration of about 20 mM to about 200 mM, about 20 mM to about 100 mM, about 100 mM, or about 180 mM.

Non-buffering salts may also be included in an aqueous pre-reaction solution or a crosslinking reaction mixture as an alternative to, or in addition, to buffering salts. Some examples may include sodium chloride, potassium chloride, potassium bromide, sodium bromide, lithium chloride, lithium bromide, sodium iodide, and potassium iodide The concentration of a non-buffering salt may vary. For example, some mixtures may have a non-buffering salt concentration in a range of about 10 mM to about 1 mM, about 30 mM to about 500 mM, or about 50 mM to about 300 mM. In some embodiments, sodium chloride may be present at a concentration in a range of about 0.5 % w/v to about 2 % about 0.9 % w/v, about 1.6 % w/v, about 20 mM to about 1 mM, about 40 mM to about 500 mM, about 50 to 300 mM, about 80 mM to about 330 mM, about 150 mM, or about 270 mM.

The pH of an aqueous pre-reaction solution may be lower than the pH of a crosslinking reaction mixture. If the salt content of the aqueous pre-reaction solution is low, the pH may be lower to enhance solubility of the hyaluronic acid and the collagen. If the salt content is higher, the pH may be higher in the aqueous pre-reaction solution. In some embodiments, the pH of the aqueous pre-reaction mixture is about 1 to about 8, about 3 to about 8, about 4 to about 6, about 4.7 to about 7.4, or about 5.4. For low salt concentrations, the pH may be about 1 to about 4 or about 1 to about 3.

In some embodiments, pH may be adjusted to neutral to allow collagen gelation or fiber formation before adding a coupling agent.

In some embodiments, the pH may be adjusted to neutral immediately prior to, around the time of, or after adding a coupling agent, such that collagen gelation is reduced or does not substantially occur.

Any water-soluble coupling agent may be used that can crosslink hyaluronic acid to collagen. Some non-limiting examples of a coupling agent include carbodiimides such as N,N'-dicyclohexylcarbodiimide (DCC), N,N'-diisopropylcarbodiimide (DIC), or 1-ethyl-3-(3-dimethylaminopropyl)carbodiimide (EDC). Carbodiimide coupling agents may facilitate ester or amide bond formation without becoming part of the linkage. However, other coupling agents that become part of the crosslinking group may be used. The concentration of a coupling agent may vary. In some embodiments, a coupling agent may be present at about 2 mM to about 150 mM, about 2 mM to about 50 mM, about 20 mM to about 100 mM, or about 50 mM. In some embodiments, the coupling agent is EDC that is present at a concentration of about 20 mM to about 100 mM, about 2 mM to about 50 mM, or about 50 mM.

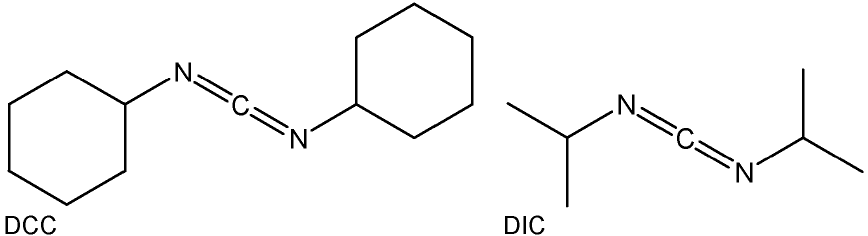

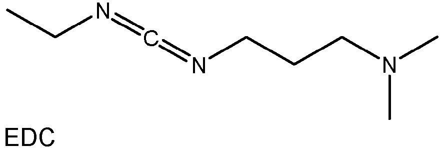

As a result of a crosslinking reaction, a crosslinked macromolecular matrix may comprise a crosslinking component that crosslinks or covalently connects the hyaluronic acid component to the collagen component. A crosslink component comprises a plurality of crosslink units, or individual covalent bonding links, between the hyaluronic acid component and the collagen component. At least a portion of the crosslink units comprise an ester bond or an amide bond. In some embodiments, at least a portion of the crosslink units may be -CON- or -CO₂-, where the N is a nitrogen from an amino acid residue.

An activating agent may be used to increase the ratio of amide bonds compared to ester bonds formed in the crosslinked product. In some embodiments, an activating agent may be a triazole such as hydroxybenzotriazole (HOBT) or 1-hydroxy-7-azabenzotriazole (HOAT); a fluorinated phenol such as pentafluorophenol; a succinimide such as N-hydroxysuccinimide (NHS) or N-hydroxysulfosuccinimide (sulfoNHS), and the like.

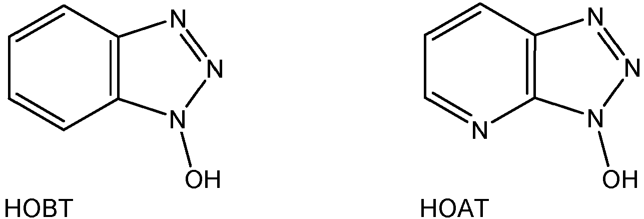

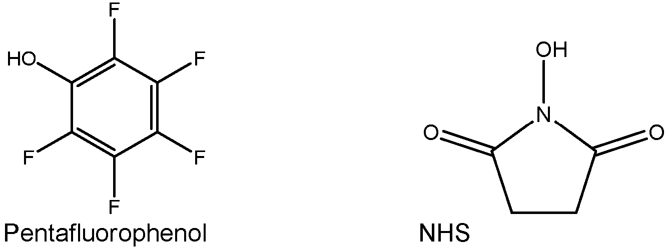

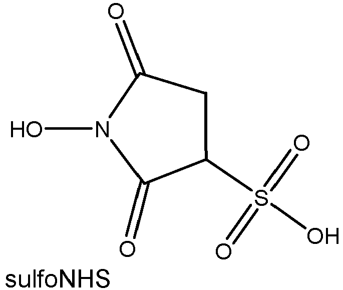

The concentration of an activating agent may vary. In some embodiments, the activating agent may have a concentration of about 2 mM to about 200 mM, about 2 mM to about 50 mM, about 20 mM to about 100 mM, or about 50 mM. In some embodments, the activating agent may be NHS or sulfoNHS is at a concentration of about 2 mM to about 50 mM. In some embodiments, the activating agent may be N-hydroxysulfosuccinimide, sodium salt, at a concentration of about 20 mM to about 100 mM, or about 50 Mm.

In some embodiments, a crosslinking reaction mixture may comprise a carbodiimide coupling agent and an activating agent. In some embodiments, the coupling agent is EDC and the activating agent is NHS or sulfoNHS. In some embodiments EDC is present at a concentration of about 2 mM to about 50 mM and NHS or sulfoNHS is present at about 2 mM to about 50 mM.

In some embodiments, a crosslinking reaction mixture may comprise hyaluronic acid at a concentration of about 1.7 mg/mL, collagen at a concentration of about 1.7 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 Mm, wherein the solution has a pH of about 5.4.

In some embodiments, a crosslinking reaction mixture may comprise hyaluronic acid at a concentration of about 6 mg/mL, collagen at a concentration of about 6 mg/mL, 2-(N-morpholino)ethanesulfonic acid at a concentration of about 180 mM, sodium chloride at a concentration of about 1.6 wt% or about 270 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 16 mg/mL of, collagen at a concentration of about 8 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of between about 4.5 and 5.5, for example, about 5.2.

In some embodiments, a crosslinking reaction mixture may comprise hyaluronic acid at a concentration of about 12 mg/mL, collagen at a concentration of about 12 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM.

In some embodiments, a crosslinking reaction mixture may comprise hyaluronic acid at a concentration of about 3 mg/mL, collagen at a concentration of about 3 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 12 mg/mL, collagen at a concentration of about 6 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 24 mg/mL, collagen at a concentration of about 12 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 1 mg/mL to about 20 mg/mL, collagen at a concentration of about 1 mg/mL to about 15 mg/mL, 2-(N-morpholino)ethanesulfonic acid at a concentration of about 20 mM to about 200 mM, sodium chloride at a concentration of about 0.5 wt% to about 2 wt% or about 80 mM to about 330 mM, 1-ethyl-3-(3-dimethylaminopropyl)carbodiimide at a concentration of about 20 mM to about 100 mM, and N-hydroxysulfosuccinimide sodium salt at a concentration of about 20 mM to about 100 mM, wherein the solution has a pH of about 4 to about 6.

### Example 1

### Method of making an injectable composition

Solutions of hyaluronic acid (HA) and collagen were produced by dissolving 15 mg of 2.0 MDa hyaluronic acid in 5 mL of human collagen(III) solution at 3 mg/mL in 0.01 N hydrochloric acid (Fibrogen). The hyaluronic acid / collagen solution was then lyophilized at -50°C and 0.02 Torr. The resulting sponges were soaked in 20 mL of ethanol:water mixture at ratios varying from 1:2 to 5:1 with 50 mM of 1-ethyl-3-(3-dimethylaminopropyl) carbodiimide and 50 mM of N-hydroxysulfosuccinimide sodium salt for 24 hrs. The crosslinked gels were then washed in 70% isopropanol / 30% water for sterilization followed by five washes in sterile phosphate buffer for purification.

### Example 2

### Method of making an injectable composition

A solution of HA at 3.4 mg/mL was created by dissolving 34 mg of 2 MDa HA in 10 mL of 100 mM MES buffer with 0.9 wt% NaCl, pH 4.7. Upon full hydration and dissolution of the HA, this solution was mixed with 10 mL of 3.4 mg/mL human collagen(III) solution in 100 mM HCl. The pH of the resulting HA/collagen(III) solution was adjusted to 5.4 with 10 mM NaOH solution. EDC (192 mg) and 217 mg of sulfoNHS (50 mM each) were added to the HA/collagen(III) solution and mixed thoroughly. The crosslinking reaction proceeded for 18 hrs before the gel was particulated through a 100 micron pore-sized mesh.

### Example 3

### Method of making an injectable composition

Rat tail collagen(I) in 0.01 N hydrochloric acid (Invitrogen) was concentrated from 5 mg/mL to 8 mg/mL using a centrifugal filtration device with 20 kDa molecular weight cutoff. HA (160 mg, 2 MDa) was added to 10 mL of the collagen solution and allowed to hydrate for 60 minutes. The solution was then homogenized by passing from syringe to syringe through a leur-leur connector. NaCl (93 mg) and 201 mg of MES were added to the solution and mixed. EDC (98 mg) and 111 mg of sulfoNHS were added to the solution and quickly mixed. Finally, 200 µL of 1 N NaOH was added to the solution which was mixed by syringe-to-syringe passing. The reaction solution was transferred to a glass vial and centrifuged for 5 min at 4000 RPM to remove air bubbles. The gel was then particulated through a 60 micron pore-sized mesh. Following sizing, the gel was sterilized by dialysis through a 20 kDa molecular-weight cut-off cellulose ester membrane against 70% isopropanol / 30% water for 3 hrs at 4°C. Dialysis was then continued against sterile phosphate buffer for 48 hrs at 4°C with three changes of buffer. The gel was then dispensed into syringes under aseptic conditions.

### Example 4

### Method of making an injectable composition

Rat tail collagen(I) in 0.01 N hydrochloric acid (Invitrogen) was concentrated from 5 mg/mL to 12 mg/mL using a centrifugal filtration device with 20 kDa molecular weight cutoff. HA (120 mg, 2 MDa) was added to 10 mL of the collagen solution and allowed to hydrate for 60 minutes. The solution was then homogenized by passing from syringe to syringe through a leur-leur connector. NaCl (93 mg) and 201 mg of MES were added to the solution and mixed. EDC (98 mg) and 111 mg of sulfoNHS were added to the solution and quickly mixed. Finally, 200 µL of 1 N NaOH was added to the solution which was mixed by syringe-to-syringe passing. The reaction solution was transferred to a glass vial and centrifuged for 5 min at 4000 RPM to remove air bubbles. The gel was then particulated through a 60 micron pore-sized mesh. Following sizing, the gel was sterilized by dialysis through a 20 kDa molecular-weight cut-off cellulose ester membrane against 70% isopropanol / 30% water for 3 hrs at 4°C. Dialysis was then continued against sterile phosphate buffer for 48 hrs at 4°C with three changes of buffer. The gel was then dispensed into syringes under aseptic conditions.

### Example 5

### Method of making an injectable composition

Rat tail collagen(I) in 0.01 N hydrochloric acid (Invitrogen) was concentrated from 5 mg/mL to 12 mg/mL using a centrifugal filtration device with 20 kDa molecular weight cutoff. HA (120 mg, 2 MDa) was added to 10 mL of the collagen solution and allowed to hydrate for 60 minutes. The solution was then homogenized by passing from syringe to syringe through a leur-leur connector. NaCl (93 mg), 201 mg of MES, and 200 µL of 1 N NaOH were added to the solution, mixed, and given 45 minutes for collagen polymerization. EDC (98 mg) and 111 mg of sulfoNHS were then added and the final solution was mixed by syringe-to-syringe passing. The reaction solution was transferred to a glass vial and centrifuged for 5 min at 4000 RPM to remove air bubbles. The gel was then particulated through a 60 micron pore-sized mesh. Following sizing, the gel was sterilized by dialysis through a 20 kDa molecular-weight cut-off cellulose ester membrane against 70% isopropanol / 30% water for 3 hrs at 4°C. Dialysis was then continued against sterile phosphate buffer for 48 hrs at 4°C with three changes of buffer. The gel was then dispensed into syringes under aseptic conditions.

### Example 6

### Rheology characterization of the compositions

Oscillatory parallel plate rheology was used to characterize the mechanical properties of gels using an Anton Paar MCR 301. A plate diameter of 25 mm was used at a gap height of 1 mm. A frequency sweep from 0.1 to 10 Hz at a fixed strain of 2% with logarithmic increase in frequency was applied followed by a strain sweep between 0.1% and 300% at a fixed frequency of 5 Hz with logarithmic increase in strain. The storage modulus (G') and loss modulus (G") were determined from frequency sweep measurements at 5 Hz.

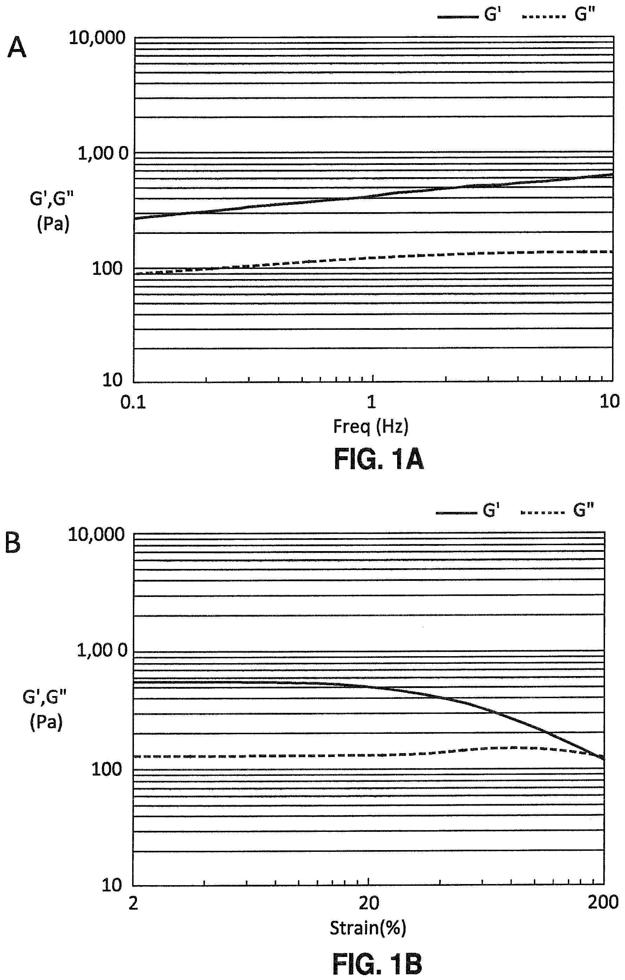

The gel from Example 4 had a storage modulus (G') of 556 Pa and loss modulus (G") of 131 Pa. The frequency sweep (A) and strain sweep (B) are shown in Figure 1.

### Example 7

### Extrusion test

In order to determine the force required to extrude the gels, they were ejected from 1 mL BD syringes through 30G needles using an Instron 5564 with Bluehill 2 software. The plunger was pushed at a rate of 100 mm/min for 11.35 mm and the extrusion profile was recorded.

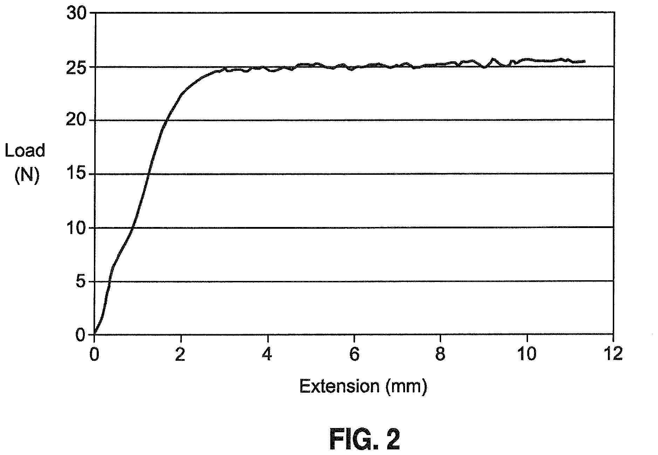

The extrusion profile through a 30G needle for gel from Example 4 is shown in Figure 2. The gel had an average extrusion force of 25 N from 4 through 10 mm.

### Example 8

### Method of making dermal fillers

Hyaluronic acid, 2 MDa molecular weight, was dissolved in human collagen(I) solution in 0.01 N hydrochloric acid (Advanced BioMatrix). Sodium chloride was added at 0.9 wt% and 2-(N-morpholino)ethanesulfonic acid was added at 100 mM to the solution and mixed. The hyaluronic acid was allowed to hydrate for 1 hr and the solution was homogenized by syringe-to-syringe mixing. The pH of the solution was adjusted to 5.4 by addition of 1 N sodium hydroxide. 1-Ethyl-3-(3-dimethylaminopropyl) carbodiimide (50 mM) and N-hydroxysulfosuccinimide sodium salt (50 mM) were added to the hyaluronic acid / collagen solution and quickly mixed by syringe-to-syringe transfer. The solution was transferred to a glass vial and centrifuged for 5 min at 4000 RPM to remove air bubbles. The resulting gel was allowed to react for 16 hrs at 4 °C. The gel was then particulated through a 100 micron pore-sized mesh. Following sizing, the gel was sterilized by dialysis through a 20 kDa molecular-weight cut-off cellulose ester membrane against 70% isopropanol / 30% water for 3 hrs at 4°C. Dialysis was then continued against sterile phosphate buffer, pH 7.4, for 48 hrs at 4°C with four changes of buffer. The gel was then dispensed into syringes under aseptic conditions.

This procedure was used to produce hydrogels with varying concentrations of hyaluronic acid and collagen. When required, human collagen(I) in 0.01 N hydrochloric acid was concentrated from 3 mg/mL to the desired reaction concentration in 20 kDa molecular-weight cut-off centrifugal filtration devices. A 50 mL sample of each gel was synthesized, sterilized by exposure to 70% isopropanol, and purified by dialysis against phosphate buffer, pH 7.4. The gels synthesized are described in Table 2 along with their rheological properties.

**Table 2: Hyaluronic acid-human collagen(I) hydrogel synthesis concentrations and rheological properties (*: comparative)**

| **Sample ID** | **[HA] (mg/mL)** | **[Col(I)] (mg/mL)** | **G' (Pa)** | **G" (Pa)** |
|---|---|---|---|---|
| **A*** | 3 | 3 | 199 | 24.6 |
| **B** | 12 | 6 | 1260 | 154 |
| **C** | 16 | 8 | 2450 | 288 |
| **D** | 12 | 12 | 3160 | 420 |
| **E** | 24 | 12 | 5440 | 433 |
| **F** | 12 | 3 | 1110 | 52.2 |
| **G** | 16 | 3 | 1490 | 60.6 |
| **H** | 20 | 3 | 1770 | 49.5 |

### Example 9

### Biopolymer concentration of the dermal fillers

In order to determine the biopolymer concentration in gels, the weight of the hydrated gel was compared to that of dried gel. A 2 mL sample of gel was weighed and dried by flash-freezing in liquid nitrogen followed by lyophilization at -50°C and 0.02 Torr. A solution of the appropriate buffer was also weighed and dried in the same fashion to account for salt content of the gel. The total solids content of the gel was calculated by dividing the dry weight by the wet volume, assuming 1 g/mL density for the wet gel, to give a value in mg/mL. The salt solids content was then subtracted from this value to determine the biopolymer concentration in the gel.

**Table 3: Final concentrations of hyaluronic acid-human collagen(I) hydrogels (*: comparative).**

| Sample ID | [HA](mg/mL) | [Col(I)] (mg/mL) | Final concentration (mg/mL) |
|---|---|---|---|
| A* | 3 | 3 | 5.3 |
| B | 12 | 6 | 16.3 |
| C | 16 | 8 | 19.4 |
| D | 12 | 12 | 22.6 |
| E | 24 | 12 | 31.6 |

### Example 10

### Swelling ratios

Swelling ratios relative to initial water content were determined for gels by increase in weight when equilibrated with phosphate buffer. For each gel, approximately 1 mL was injected into a 15 mL Falcon tube and weighed, followed by addition of 10 mL of phosphate buffered saline, pH 7.4. The gels were thoroughly mixed with the buffer and vortexed for 30 seconds. The gels were then allowed to equilibrate in the buffer for 48 hrs at 4°C. After this time, the suspensions were centrifuged at 4000 RPM in a swinging bucket rotor for 5 minutes. The supernatant buffer was then decanted and the weight of the swollen gel was measured. The swelling ratio was determined by dividing the final weight of the swollen gel by the weight of the initial gel.

**Table 4: Swelling ratios of hyaluronic acid-human collagen(I) hydrogels (comparative)**

| Sample ID | [HA] (mg/mL) | [Col(I)] (mg/mL) | Swelling ratio |
|---|---|---|---|
| A* | 3 | 3 | 0.96 |
| B | 12 | 6 | 1.67 |
| C | 16 | 8 | 1.69 |
| D | 12 | 12 | 1.49 |
| E | 24 | 12 | 1.65 |

### Example 11

### HA/Collagen for Facial Defects of Check

This example illustrates the use of compositions and methods disclosed herein for a facial disorder.

A 58-year-old woman presented with a lean face. She felt her face looked old, sad and bitter because of the less fullness of her cheek contour. Pre-operative evaluation of the person includes routine history and physical examination in addition to thorough informed consent disclosing all relevant risks and benefits of the procedure. The physician evaluating the individual determines that she is a candidate for administration of the dermal filler compositions and methods disclosed herein.

A composition of the invention, such as described in EXAMPLE 4, is provided in a 20 mL syringe. One-holed blunt infiltration cannulas (3 mm inner diameter) are used to place about 15 mL of the composition in the syringe subcutaneously and under superficial musculoaponeurotix system into the left and right checks.

The individual is monitored for approximately 7 days. The physician evaluates the treatment area and determines that the treatment was successful. The woman's cheeks are fuller than prior to treatment, Both the woman and her physician are satisfied with the results of the procedure because she looks younger than she did when she came in for treatment.

### Example 12

### Treatment of Facial Defects of Eyelids

This example illustrates the use of compositions and methods disclosed herein for a treatment of eyelid defects.

A 37-year-old woman presented with fine wrinkles around her eyes and she reports that her eyes made her look old and angry. Pre-operative evaluation of the person includes routine history and physical examination in addition to thorough informed consent disclosing all relevant risks and benefits of the procedure. The physician evaluating the individual determines that she is a candidate for administration of the dermal filler compositions and methods disclosed herein.

A composition, such as made as described in Example 5, is provided in a 20 mL syringe. About 2.5 mL of the composition is injected with a fine needle subcutaneously in the skin beneath the wrinkles into the regions adjacent the eyes.

The individual is monitored for approximately 7 days. The physician evaluates the eye of the patient and determines that the treatment was successful. Both the woman and her physician are satisfied with the results of the procedure because her eyes appear refreshed and the skin appears rejuvenated. Approximately one year after the procedure, the woman indicates that her quality of life has improved.

### Example 13

### Treatment of acne scars

This example illustrates the use of compositions and methods disclosed herein for treatment of acne scars.

A 25-year-old man presents with moderate acne scarring on his jaw line including depressions and pitting. He reports that he is dissatisfied with his appearance and feels he is socially inhibited due to his perception of his appearance. Pre-operative evaluation of the person includes routine history and physical examination in addition to thorough informed consent disclosing all relevant risks and benefits of the procedure. The physician evaluating the individual determines that he is a candidate for administration of the dermal filler compositions and methods disclosed herein.

A composition, such as that made as described in Example 12, is provided in 10 mL syringes. The physician injects a small amount of the composition below the skin in each depressed or pitted area of the patient's jawline to raise the area to match the surrounding skin.

The individual returns for a follow up visit with the physician in 14 days. The physician evaluates the patient and determines that the treatment was successful. The man reports he is satisfied with the results of the procedure because his skin is more smooth in appearance and the acne scarring is substantially less visible. Approximately six months after the procedure, the man returns for a follow up treatment. He reports to the physician that his quality of life has greatly improved since the procedure and he is no longer shy about his appearance.

## Claims

1. A soft tissue aesthetic product comprising:
a filler comprising a homogeneous hydrogel having a form suitable for injecting into mammalian tissue; and
a label comprising instructions to inject the filler into the tissue;
wherein the homogeneous hydrogel comprises water, and a crosslinked macromolecular matrix comprising:
a hyaluronic acid component; and
a collagen component;
wherein the hyaluronic acid component is crosslinked to the collagen component by a crosslinking component; and
wherein the crosslinking component comprises a plurality of crosslink units,
wherein at least a portion of the crosslink units comprise an ester bond or an amide bond
wherein the hyaluronic acid concentration is from 6 mg/ml to 24 mg/ml; the collagen concentration is from 1 mg/ml to 15 mg/ml; and the hydrogel has a storage modulus value of between 50 Pa and 10,000 Pa.

2. The product of claim 1, wherein the collagen component comprises collagen type I or collagen type III.

3. The product of claim 1, wherein crosslinked macromolecular matrix has a weight ratio of the hyaluronic acid component to the collagen component of about 1 to about 7.

4. The product of claim 1, wherein the hydrogel is in the form of a dermal filler.

5. The product of claim 1, wherein the hydrogel is in the form of a lip filler.

6. A method of improving an aesthetic quality of soft tissue of a human being comprising:
injecting a homogeneous hydrogel composition into a soft tissue of the human being to thereby improve the aesthetic quality of the soft tissue;
wherein the homogeneous hydrogel composition comprises water, and a crosslinked macromolecular matrix comprising:
a hyaluronic acid component; and
a collagen component;
wherein the hyaluronic acid component is crosslinked to the collagen component by a crosslinking component; and
wherein the crosslinking component comprises a plurality of crosslink units, wherein at least a portion of the crosslink units comprise an ester bond or an amide bond,
wherein the hyaluronic acid concentration is from 6 mg/ml to 24 mg/ml; the collagen concentration is from 1 mg/ml to 15 mg/ml; and the hydrogel has a storage modulus value of between 50 Pa and 10,000 Pa.

7. The method of claim 6, wherein the collagen component comprises collagen type I or collagen type III.

8. The method of claim 6, wherein the hydrogel is in the form of a dermal filler.

9. The method of claim 6, wherein the hydrogel is in the form of a lip filler.

10. A dermal filler comprising:
a homogeneous hydrogel having a form suitable for injecting into mammalian tissue;
wherein the homogeneous hydrogel comprises water, and a crosslinked macromolecular matrix comprising:
a hyaluronic acid component; and
a collagen component;
wherein the hyaluronic acid component is crosslinked to the collagen component by a crosslinking component; and
wherein the crosslinking component comprises a plurality of crosslink units,
wherein at least a portion of the crosslink units comprise an ester bond or an amide bond,
wherein the hyaluronic acid concentration is from 6 mg/ml to 24 mg/ml; the collagen concentration is from 1 mg/ml to 15 mg/ml; and the hydrogel has a storage modulus value of between 50 Pa and 10,000 Pa.

11. The dermal filler of claim 10, wherein the collagen component comprises collagen type I or collagen type III.

12. The dermal filler of claim 10, wherein the crosslinked macromolecular matrix has a weight ratio of the hyaluronic acid component to the collagen component of about 1 to about 7.

13. The product of claims 1 - 5, the method of claims 6 - 9, or the dermal filler of claims 10 - 12, wherein the hyaluronic acid concentration and the collagen concentration is in an aqueous pre-reaction solution or a crosslinking reaction mixture.

## Patentansprüche

1. Weichgewebe-Ästhetikprodukt, umfassend:
einen Filler umfassend ein homogenes Hydrogel das eine zum Injizieren in Säugetier-Gewebe geeignete Form aufweist; und
ein Etikett, das Anweisungen zum Injizieren des Fillers in das Gewebe umfasst;
wobei das homogene Hydrogel umfasst Wasser und eine vernetzte makromolekulare Matrix umfassend:
eine Hyaluronsäurekomponente; und
eine Kollagenkomponente;
wobei die Hyaluronsäurekomponente mit der Kollagenkomponente mittels einer vernetzenden Komponente vernetzt ist; und
wobei die vernetzende Komponente umfasst eine Mehrzahl an Vernetzungseinheiten,
wobei wenigstens ein Abschnitt der Vernetzungseinheiten eine Esterbindung oder eine Amidbindung umfasst,
wobei die Hyaluronsäurekonzentration von 6 mg/ml bis 24 mg/ml beträgt; die Kollagenkonzentration von 1 mg/ml bis 15 mg/ml beträgt; und das Hydrogel einen Speichermodulwert von zwischen 50 Pa und 10.000 Pa hat.

2. Produkt gemäß Anspruch 1, wobei die Kollagenkomponente Kollagen Typ I oder Kollagen Typ III umfasst.

3. Produkt gemäß Anspruch 1, wobei die vernetzte makromolekulare Matrix ein Gewichtsverhältnis der Hyaluronsäurekomponente zu der Kollagenkomponente von etwa 1 bis etwa 7 aufweist.

4. Produkt gemäß Anspruch 1, wobei das Hydrogel in der Form eines Dermalfillers ist.

5. Produkt gemäß Anspruch 1, wobei das Hydrogel in der Form eines Lippenfillers ist.

6. Verfahren zur Verbesserung einer ästhetischen Eigenschaft eines Weichgewebes eines menschlichen Wesens, umfassend:
Injizieren einer homogenen Hydrogelzusammensetzung in ein Weichgewebe des menschlichen Wesens, wodurch die ästhetische Eigenschaft des Weichgewebes verbessert wird;
wobei die homogene Hydrogelzusammensetzung umfasst Wasser und eine vernetzte makromolekulare Matrix umfassend:
eine Hyaluronsäurekomponente; und
eine Kollagenkomponente;
wobei die Hyaluronsäurekomponente mit der Kollagenkomponente mittels einer vernetzenden Komponente vernetzt ist; und
wobei die vernetzende Komponente eine Mehrzahl an Vernetzungseinheiten umfasst, wobei wenigstens ein Abschnitt der Vernetzungseinheiten eine Esterbindung oder eine Amidbindung umfasst,
wobei die Hyaluronsäurekonzentration von 6 mg/ml bis 24 mg/ml beträgt; die Kollagenkonzentration von 1 mg/ml bis 15 mg/ml beträgt; und das Hydrogel einen Speichermodulwert von zwischen 50 Pa und 10.000 Pa hat.

7. Verfahren gemäß Anspruch 6, wobei die Kollagenkomponente Kollagen Typ I oder Kollagen Typ III umfasst.

8. Verfahren gemäß Anspruch 6, wobei das Hydrogel in der Form eines Dermalfillers ist.

9. Verfahren gemäß Anspruch 6, wobei das Hydrogel in der Form eines Lippenfillers ist.

10. Dermalfiller, umfassend:
ein homogenes Hydrogel, das eine zum Injizieren in Säugetier-Gewebe geeignete Form aufweist;
wobei das homogene Hydrogel umfasst Wasser und eine vernetzte makromolekulare Matrix umfassend:
eine Hyaluronsäurekomponente; und
eine Kollagenkomponente;
wobei die Hyaluronsäurekomponente mit der Kollagenkomponente mittels einer vernetzenden Komponente vernetzt ist; und
wobei die vernetzende Komponente eine Mehrzahl an Vernetzungseinheiten umfasst,
wobei wenigstens ein Abschnitt der Vernetzungseinheiten eine Esterbindung oder eine Amidbindung umfasst,
wobei die Hyaluronsäurekonzentration von 6 mg/ml bis 24 mg/ml beträgt; die Kollagenkonzentration von 1 mg/ml bis 15 mg/ml beträgt; und das Hydrogel einen Speichermodulwert von zwischen 50 Pa und 10.000 Pa hat.

11. Dermalfiller gemäß Anspruch 10, wobei die Kollagenkomponente Kollagen Typ I oder Kollagen Typ III umfasst.

12. Dermalfiller gemäß Anspruch 10, wobei die vernetzte makromolekulare Matrix ein Gewichtsverhältnis der Hyaluronsäurekomponente zu der Kollagenkomponente von etwa 1 bis etwa 7 aufweist.

13. Produkt gemäß den Ansprüchen 1-5, Verfahren gemäß den Ansprüchen 6-9 oder Dermalfiller gemäß den Ansprüchen 10-12, wobei die Hyaluronsäurekonzentration und die Kollagenkonzentration in einer wässrigen Vorreaktionslösung oder einer Vernetzungsreaktionsmischung ist.

## Revendications

1. Produit d'esthétique pour tissu mou comprenant :
un agent de comblement comprenant un hydrogel homogène présentant une forme appropriée pour injection dans un tissu de mammifère ; et
une étiquette comprenant des instructions pour injecter l'agent de comblement dans le tissu ;
dans lequel l'hydrogel homogène comprend de l'eau, et une matrice macromoléculaire réticulée comprenant :
un composant d'acide hyaluronique ; et
un composant de collagène ;
dans lequel le composant d'acide hyaluronique est réticulé au composant de collagène par un composant de réticulation ; et
dans lequel le composant de réticulation comprend une pluralité d'unités de réticulation,
dans lesquelles au moins une partie des unités de réticulation comprend une liaison ester ou une liaison amide
dans lequel la concentration d'acide hyaluronique est de 6 mg/ml à 24 mg/ml ; la concentration de collagène est de 1 mg/ml à 15 mg/ml ; et l'hydrogel présente une valeur de module de conservation entre 50 Pa et 10 000 Pa.

2. Produit selon la revendication 1, dans lequel le composant de collagène comprend du collagène de type I ou du collagène de type III.

3. Produit selon la revendication 1, dans lequel la matrice macromoléculaire réticulée présente un rapport pondéral du composant d'acide hyaluronique au composant de collagène d'environ 1 à environ 7.

4. Produit selon la revendication 1, dans lequel l'hydrogel est sous la forme d'un agent de comblement dermique.

5. Produit selon la revendication 1, dans lequel l'hydrogel est sous la forme d'un agent de comblement pour lèvres.

6. Procédé d'amélioration d'une qualité esthétique d'un tissu mou d'un être humain comprenant :
une injection d'une composition d'hydrogel homogène dans un tissu mou de l'être humain pour ainsi améliorer la qualité esthétique du tissu mou ;
dans lequel la composition d'hydrogel homogène comprend de l'eau, et une matrice macromoléculaire réticulée comprenant :
un composant d'acide hyaluronique ; et
un composant de collagène ;
dans lequel le composant d'acide hyaluronique est réticulé au composant de collagène par un composant de réticulation ; et
dans lequel le composant de réticulation comprend une pluralité d'unités de réticulation, dans lequel au moins une partie des unités de réticulation comprend une liaison ester ou une liaison amide,
dans lequel la concentration d'acide hyaluronique est de 6 mg/ml à 24 mg/ml ; la concentration de collagène est de 1 mg/ml à 15 mg/ml ; et l'hydrogel présente une valeur de module de conservation entre 50 Pa et 10 000 Pa.

7. Procédé selon la revendication 6, dans lequel le composant de collagène comprend du collagène de type I ou du collagène de type III.

8. Procédé selon la revendication 6, dans lequel l'hydrogel est sous la forme d'un agent de comblement dermique.

9. Procédé selon la revendication 6, dans lequel l'hydrogel est sous la forme d'un agent de comblement pour lèvres.

10. Agent de comblement comprenant :
un hydrogel homogène présentant une forme appropriée pour injection dans un tissu de mammifère ;
dans lequel l'hydrogel homogène comprend de l'eau, et une matrice macromoléculaire réticulée comprenant :
un composant d'acide hyaluronique ; et
un composant de collagène ;
dans lequel le composant d'acide hyaluronique est réticulé au composant de collagène par un composant de réticulation ; et
dans lequel le composant de réticulation comprend une pluralité d'unités de réticulation,
dans lequel au moins une partie des unités de réticulation comprend une liaison ester ou une liaison amide,
dans lequel la concentration d'acide hyaluronique est de 6 mg/ml à 24 mg/ml ; la concentration de collagène est de 1 mg/ml à 15 mg/ml ; et l'hydrogel présente une valeur de module de conservation entre 50 Pa et 10 000 Pa.

11. Agent de comblement selon la revendication 10, dans lequel le composant de collagène comprend du collagène de type I ou du collagène de type III.

12. Agent de comblement dermique selon la revendication 10, dans lequel la matrice macromoléculaire réticulée présente un rapport en poids du composant d'acide hyaluronique au composant de collagène d'environ 1 à environ 7.

13. Produit selon les revendications 1 à 5, procédé selon les revendications 6 à 9, ou agent de comblement selon les revendications 10 à 12, dans lesquels la concentration d'acide hyaluronique et la concentration de collagène est dans une solution de pré-réaction aqueuse ou un mélange de réaction de réticulation.
